# EUROPEAN PATENT APPLICATION

(11) **EP 4 751 745 A1**
(43) Date of publication of application: **03.06.2026**
(21) Application number: 24862719.2
(22) Date of filing: 30.08.2024
(51) Int. Cl.: A61M 1/16, A61M 1/28

(54) **COMPUTER PROGRAM, IMAGE OUTPUT METHOD, AND IMAGE OUTPUT DEVICE**

(30) Priority: 06.09.2023 JP 2023144748
(71) Applicant: Terumo Kabushiki Kaisha, Tokyo 151-0072 (JP)
(72) Inventor: TAKAHASHI, Hiroaki, Ashigarakami-gun, Kanagawa 259-0151 (JP); KAWAMOTO, Eiji, Ashigarakami-gun, Kanagawa 259-0151 (JP)
(74) Representative: KIPA AB
(86) International application number: PCT/JP2024/031183
(87) International publication number: WO 2025/053067

(57) **Abstract**

A computer program, an image output method, and an image output device are provided.

A computer is caused to execute processing comprising: acquiring information on a peritoneal dialysis patient, the information including information on at least one of edema, a urine output, and a net ultrafiltration; generating an image representing a condition of a body fluid of the peritoneal dialysis patient on a basis of the information thus acquired; and outputting the image thus generated.

## Description

### Technical Field

The present disclosure relates to a computer program, an image output method, and an image output device.

### Background Art

Conventionally, there have been disclosures of various techniques for detecting conditions of a patient in dialysis treatment. For example, Patent Literature discloses a technique of dividing a period of dialysis treatment into a plurality of periods and measuring the blood pressure of a patient in each period.

### Citation List

### Patent Literature

Patent Literature 1: JP 2000-000217 A

### Summary of Invention

### Technical Problem

Patent Literature 1 only deals with the management of the transition in the blood pressure during the period of dialysis, and fails to enable peritoneal dialysis patients and medical workers to get a grasp of the conditions of body fluids of the patients themselves intuitively.

In one aspect, an object is to provide a computer program, an image output method, and an image output device enabling intuitive understanding of the conditions of a body fluid of a peritoneal dialysis patient.

### Solution to Problem

(1) A computer program according to the present disclosure is a computer program for causing a computer to execute processing of: acquiring information on a peritoneal dialysis patient, the information including information on at least one of edema, a urine output, and a net ultrafiltration; generating an image representing a condition of a body fluid of the peritoneal dialysis patient on a basis of the information thus acquired; and outputting the image thus generated.
(2) In the computer program according to (1), preferably, the information further includes information related to at least one of a human atrial natriuretic peptide (hANP), a cardio-thoracic ratio, a bioelectrical impedance, a transperitoneal sodium removal, and a urinary sodium excretion.
(3) In the computer program according to (1) or (2), preferably, the information further includes information related to at least one of a body weight, mouth dryness, an antihypertensive agent, a diuretic, and a blood pressure of the peritoneal dialysis patient.
(4) In the computer program according to any one of (1) to (3), preferably, the information further includes information related to at least one of urinary cortisol, urinary creatinine, a skin sodium content, and nuclear magnetic resonance (Na-MRI).
(5) In the computer program according to any one of (1) to (4), preferably, an image schematically representing a sodium retention and a sodium excretion of the peritoneal dialysis patient is generated as the image.
(6) In the computer program according to any one of (1) to (5), preferably, an image including a first vessel storing interstitial fluid and a second vessel storing plasma, and representing the sodium retention as an amount of interstitial fluid in the first vessel is generated as the image.
(7) In the computer program according to any one of (1) to (6), an image including a passage through which the interstitial fluid and the plasma flow between the first vessel and the second vessel, and an excretion path through which sodium is excreted as urine from the second vessel is generated as the image, the image being an image schematically representing the sodium excretion on a basis of an amount of sodium removed from the plasma by peritoneal dialysis and the amount of sodium excreted through the excretion path.
(8) In the computer program according to any one of (1) to (7), preferably, an image schematically representing a balance between a sodium intake and a sodium excretion of the peritoneal dialysis patient is generated, as the image.
(9) In the computer program according to any one of (1) to (8), preferably, acquired information on the peritoneal dialysis patient is input to a learning model having been trained to output information on a condition of the body fluid in response to an input of the information on at least one of edema, a urine output, and a net ultrafiltration; the learning model is caused to execute computation; and an image representing the condition of the body fluid of the peritoneal dialysis patient is generated on a basis of a result of the computation of the learning model.
(10) An image output method according to the present disclosure uses a computer to execute processing including: acquiring information on a peritoneal dialysis patient, the information including information on at least one of edema, a urine output, and a net ultrafiltration; generating an image representing a condition of a body fluid of the peritoneal dialysis patient on a basis of the information thus acquired; and outputting the image thus generated.
(11) An image output device according to the present disclosure includes at least one processor, and the processor acquires information on a peritoneal dialysis patient, the information including information on at least one of edema, a urine output, and a net ultrafiltration; generates an image representing a condition of a body fluid of the peritoneal dialysis patient on a basis of the information thus acquired; and outputs the image thus generated.

### Advantageous Effects of Invention

According to one aspect, the conditions of a body fluid of a peritoneal dialysis patient can be understood intuitively.

### Brief Description of Drawings

[Fig. 1] Fig. 1 is a schematic illustrating a configuration example of a body fluid management support system according to an embodiment.
[Fig. 2] Fig. 2 is a block diagram illustrating an internal configuration of a server device.
[Fig. 3] Fig. 3 is a block diagram illustrating an internal configuration of a patient's terminal.
[Fig. 4] Fig. 4 is a block diagram illustrating an internal configuration of a doctor's terminal.
[Fig. 5] Fig. 5 is a schematic illustrating an example of a patient information input screen on the patient's terminal.
[Fig. 6] Fig. 6 is a schematic illustrating an example of a patient information input screen on the doctor's terminal.
[Fig. 7] Fig. 7 is a schematic illustrating a configuration example of a learning model.
[Fig. 8A] Fig. 8A is a schematic illustrating an example of a generated image.
[Fig. 8B] Fig. 8B is a schematic illustrating an example of a generated image.
[Fig. 8C] Fig. 8C is a schematic illustrating an example of a generated image.
[Fig. 9A] Fig. 9A is a schematic illustrating a modification of an image generated by the server device.
[Fig. 9B] Fig. 9B is a schematic illustrating a modification of an image generated by the server device.
[Fig. 10] Fig. 10 is a flowchart for explaining the sequence of processing executed by the server device according to this embodiment.

### Description of Embodiments

The present invention will now be described in detail, with reference to drawings illustrating an embodiment of the present invention.

### (First Embodiment)

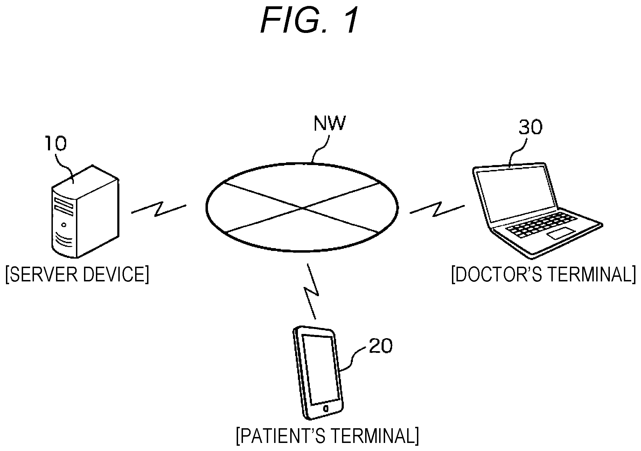

Fig. 1 is a schematic illustrating a configuration example of a body fluid management support system according to an embodiment. The body fluid management support system according to the embodiment includes a server device 10, a patient's terminal 20, and a doctor's terminal 30. The server device 10 is, for example, a server computer installed by a hospital. The patient's terminal 20 is a client computer to be used by a peritoneal dialysis patient (hereinafter, also simply referred to as a patient), and the doctor's terminal 30 is a client computer to be used by a medical worker such as a doctor. The server device 10, the patient's terminal 20, and the doctor's terminal 30 are communicably connected to one another over a communication network NW.

The server device 10 acquires information on the peritoneal dialysis patient (hereinafter, referred to as patient information) from the patient's terminal 20 or the doctor's terminal 30, and generates an image representing the condition of a body fluid of the peritoneal dialysis patient, on the basis of the acquired patient information.

The patient information acquired by the server device 10 includes information related to at least one of edema, a urine output, and a net ultrafiltration. The information related to edema, a urine output, and a net ultrafiltration can be received via the patient's terminal 20. The patient information acquired by the server device 10 may further include information such as that of mouth dryness and a body weight entered via the patient's terminal 20.

The patient information acquired by the server device 10 may further include information related to at least one of hANP, a cardio-thoracic ratio, a bioelectrical impedance, a transperitoneal sodium removal, and a urinary sodium excretion. These pieces of information are information managed per patient, and may be input from the doctor's terminal 30 and registered in the server device 10 in advance, for example. The patient information registered in the server device 10 may further include information related to an antihypertensive agent, a diuretic, a blood pressure, or the like, and may further include information related to urinary cortisol, urinary creatinine, a skin sodium content, Na-MRI, or the like.

The server device 10 outputs an image generated on the basis of the patient information. For example, the server device 10 may present the condition of the body fluid to the patient, by transmitting the generated image to the patient's terminal 20 via the communication network NW. The patient can thus intuitively understand the condition of his/her body fluid. Alternatively, the server device 10 may transmit the generated image to the doctor's terminal 30 via the communication network NW, and present the condition of the body fluid of the patient to the medical worker. The medical worker can get a grasp of the condition of the body fluid of the patient, and use the information as a community tool for giving instructions on appropriate body fluid management.

An internal configuration of each of the devices included in the body fluid management support system will now be described.

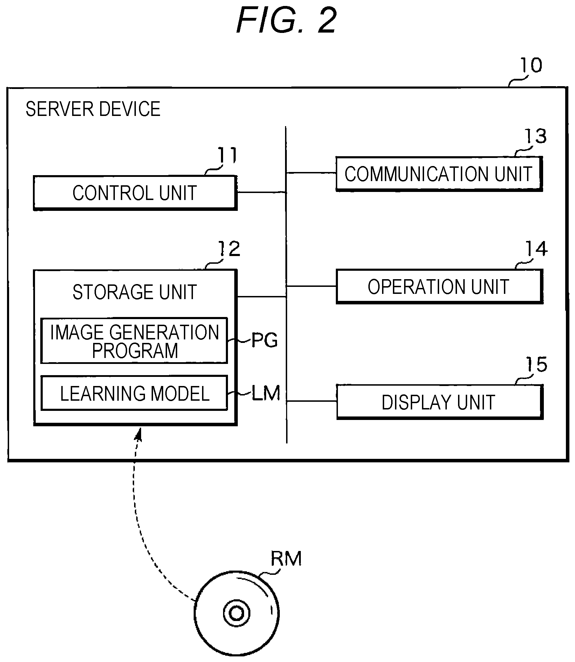

Fig. 2 is a block diagram illustrating an internal configuration of the server device 10. The server device 10 is a dedicated or general-purpose computer, and includes a control unit 11, a storage unit 12, a communication unit 13, an operation unit 14, and a display unit 15.

The control unit 11 includes, for example, a central processing unit (CPU), a read-only memory (ROM), and a random access memory (RAM). The ROM included in the control unit 11 stores a control program and the like for controlling the operation of each piece of hardware included in the server device 10. The CPU in the control unit 11 causes the entire device to function as the server device 10 (image output device) according to the present disclosure, by reading and executing a control program stored in the ROM, or a computer program, which will be described later, stored in the storage unit 12, and by controlling the operation of each piece of hardware. In the RAM included in the control unit 11, data used during the execution of computation or control is temporarily stored.

In the embodiment, the control unit 11 includes the CPU, the ROM, and the RAM, but the configuration of the control unit 11 is not limited to the one described above. For example, the control unit 11 may be one or more control circuits or arithmetic circuits including a graphics processing unit (GPU), a field-programmable gate array (FPGA), a digital signal processor (DSP), a quantum processor, and a volatile or nonvolatile memory. The control unit 11 may also have functions such as a clock that outputs the time and date information, a timer that measures the time elapsed from when a measurement start instruction is given to when a measurement end instruction is given, a counter that increments a number.

The storage unit 12 includes a storage device such as a hard disk drive (HDD) or a solid-state drive (SSD). The storage unit 12 stores therein various computer programs to be executed by the control unit 11 and various types of data to be used by the control unit 11.

A computer program (program product) stored in the storage unit 12 includes an image generation program PG for causing a computer to execute processing of acquiring information on a peritoneal dialysis patient, the information including information of at least one of edema, a urine output, and a net ultrafiltration, of generating an image representing the conditions of a body fluid of the peritoneal dialysis patient on the basis of the acquired information, and of outputting the generated image. The image generation program PG may be a single computer program or a program set including a plurality of computer programs. The image generation program PG may use a part of an existing library. The image generation program PG may be executed by a single computer or may be executed by a plurality of computers in a cooperative association.

The computer program including the image generation program PG is provided via a non-transitory recording medium RM in which the computer program is readably recorded. The recording medium RM is a portable memory such as a CD-ROM, a USB memory, or a secure digital (SD) card. The control unit 11 reads various computer programs from the recording medium RM using a reader, not illustrated, and stores the read various computer programs in the storage unit 12. The computer program including the image generation program PG may also be provided by communication. The control unit 11 acquires a computer program including the image generation program PG by communication via the communication unit 13, and stores the acquired computer program in the storage unit 12.

The storage unit 12 may also store therein a learning model LM having been trained to output information related to the condition of a body fluid in response to an input of the information related to at least one of edema, a urine output, and a net ultrafiltration. In relation to the learned learning model LM, the storage unit 12 stores information describing the learning model LM such as the layer structure of the model, the number of nodes included in each layer, connection relationships, weighting coefficients and biases among the nodes, and learned parameters.

The communication unit 13 includes a communication interface via which various types of data are transmitted to and received from an external device. As the communication interface of the communication unit 13, a communication interface conforming to a communication protocol such as WiFi (registered trademark), local area network (LAN), Bluetooth (registered trademark), ZigBee (registered trademark), 3G, 4G, 5G, or the long-term evolution (LTE) may be used. Upon receiving data to be transmitted from the control unit 11, the communication unit 13 transmits the data to the destination external device. Upon receiving data from an external device, the communication unit 13 outputs the received data to the control unit 11. In this embodiment, examples of the external device are the patient's terminal 20 and the doctor's terminal 30.

The operation unit 14 includes an operation device such as a touch panel, a keyboard, and a switch, and receives various operations and settings from a user or the like. The control unit 11 performs appropriate control on the basis of various operation information given via the operation unit 14, and stores the setting information in the storage unit 12, as necessary.

The display unit 15 includes a display device such as a liquid crystal display or an organic electro-luminescence (EL) display, and displays information to be presented, in response to an instruction from the control unit 11.

In this embodiment, the server device 10 may be a single computer, or may be a computer system including a plurality of computers, peripheral devices, and the like. Furthermore, the server device 10 may be a virtual machine that is a virtualized entity, or may be a cloud.

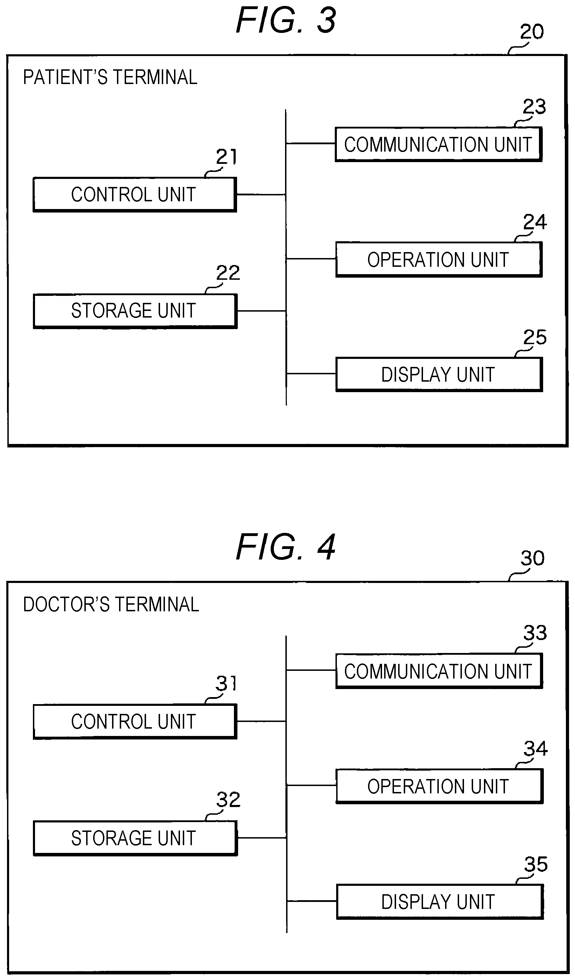

Fig. 3 is a block diagram illustrating an internal configuration of the patient's terminal 20. The patient's terminal 20 is a computer such as a smartphone, a tablet terminal, or a personal computer, and includes a control unit 21, a storage unit 22, a communication unit 23, an operation unit 24, and a display unit 25.

The control unit 21 includes, for example, a CPU, a ROM, a RAM, and the like. The CPU of the control unit 21 controls the operations of various pieces of hardware described above to cause the entire device to function as the patient's terminal 20 in the present disclosure by loading various programs stored in advance in the ROM or the storage unit 22 onto the RAM, and executing the programs.

Note that the control unit 21 is not limited to the configuration described above, and may be configured as a single piece of hardware in which a processor, a memory, a storage, a communication interface, and the like are integrated (system on a chip (SoC)). The control unit 21 may also have functions such as a clock that outputs the time and date information, a timer that measures the time elapsed from when a measurement start instruction is given to when a measurement end instruction is given, and a counter that increments a number.

The storage unit 22 includes a storage such as a memory or a hard disk. The storage unit 22 stores therein various computer programs to be executed by the control unit 21, data required for executing the computer programs, and the like. The computer programs stored in the storage unit 22 include an application program for making use of a service provided by the server device 10.

Note that the programs to be stored in the storage unit 22 may also be provided via a non-transitory recording medium in which the program is readably recorded. The recording medium is a portable memory such as a CD-ROM, a USB memory, or a SD card, for example. The control unit 21 reads various programs from the recording medium using a reader, not illustrated, and installs the read various programs in the storage unit 22. The programs to be stored in the storage unit 22 may be provided by communication. In such a case, the control unit 21 acquires the various programs via the communication unit 23, and installs the acquired various programs in the storage unit 22.

The communication unit 23 includes a communication interface for establishing a connection to the communication network NW. The communication interface included in the communication unit 23 is a communication interface conforming to a communication protocol such as WiFi (registered trademark), LAN, Bluetooth (registered trademark), ZigBee (registered trademark), 3G, 4G, 5G, or LTE, for example. The communication unit 23 transmits various types of information to be notified to the external, and receives various types of information transmitted to the patient's terminal 20 from the external.

The operation unit 24 includes an input device such as a touch panel or an operation button, and receives various operation information and setting information. The operation unit 24 outputs the received operation information and setting information to the control unit 21. The control unit 21 performs appropriate control on the basis of the operation information input via the operation unit 24, and stores the setting information in the storage unit 22, as necessary.

The display unit 25 includes a display device such as a liquid-crystal display or an organic EL display, and displays information to be presented to the patient on the basis of a control signal output from the control unit 21.

Fig. 4 is a block diagram illustrating an internal configuration of the doctor's terminal 30. The doctor's terminal 30 is a computer such as a personal computer, a tablet terminal, or a smartphone, and includes a control unit 31, a storage unit 32, a communication unit 33, an operation unit 34, and a display unit 35.

The control unit 31 includes, for example, a CPU, a ROM, a RAM, and the like. The CPU of the control unit 31 controls the operations of various pieces of hardware described above to cause the entire device to function as the doctor's terminal 30 in the present disclosure by loading various programs stored in advance in the ROM or the storage unit 32 onto the RAM, and executing the programs.

Note that the control unit 31 is not limited to the configuration described above, and may be configured as a single piece of hardware in which a processor, a memory, a storage, a communication interface, and the like are integrated (system on a chip (SoC)). The control unit 31 may also have functions such as a clock that outputs the time and date information, a timer that measures the time elapsed from when a measurement start instruction is given to when a measurement end instruction is given, and a counter that increments a number.

The storage unit 32 includes a storage such as a memory or a hard disk. The storage unit 32 stores therein various computer programs to be executed by the control unit 31, data required for executing the computer programs, and the like. The computer programs stored in the storage unit 32 include an application program for making use of a service provided by the server device 10.

Note that the programs to be stored in the storage unit 32 may also be provided via a non-transitory recording medium in which the program is readably recorded. The recording medium is a portable memory such as a CD-ROM, a USB memory, or a SD card, for example. The control unit 31 reads various programs from the recording medium using a reader, not illustrated, and installs the read various programs in the storage unit 32. The programs to be stored in the storage unit 32 may be provided by communication. In such a case, the control unit 31 acquires the various programs via the communication unit 33, and installs the acquired various programs in the storage unit 32.

The communication unit 33 includes a communication interface for establishing a connection to the communication network NW. The communication interface included in the communication unit 33 is a communication interface conforming to a communication protocol such as WiFi (registered trademark), LAN, Bluetooth (registered trademark), ZigBee (registered trademark), 3G, 4G, 5G, or LTE, for example. The communication unit 33 transmits various types of information to be notified to the external, and receives various types of information transmitted to the doctor's terminal 30 from the external.

The operation unit 34 includes an input device such as a keyboard and a mouse, and receives various operation information and setting information. The operation unit 34 outputs the received operation information and setting information to the control unit 31. The control unit 31 performs appropriate control on the basis of the operation information input via the operation unit 34, and stores the setting information in the storage unit 32, as necessary.

The display unit 35 includes a display device such as a liquid-crystal display or an organic EL display, and displays information to be presented to a medical worker on the basis of a control signal output from the control unit 31.

An operation of the body fluid management support system will now be described.

To display the condition of a body fluid of the patient himself/herself on the display unit 25 of the patient's terminal 20 using the body fluid management support system according to this embodiment, the patient inputs his/her patient information via the operation unit 24 of the patient's terminal 20.

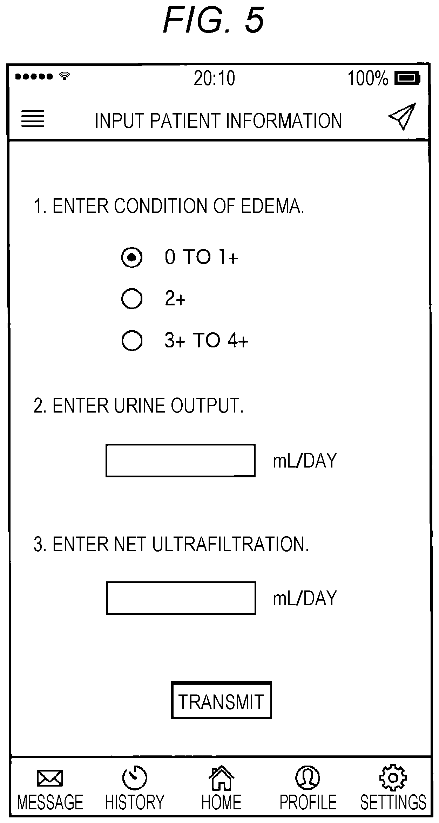

Fig. 5 is a schematic illustrating an example of a patient information input screen on the patient's terminal 20. The patient information input screen illustrated in Fig. 5 illustrates an example of a screen displayed on the display unit 25 when there is an access to the server device 10 from the patient's terminal 20 and a predetermined operation is performed. The patient information input screen receives information related to edema, a urine output, and a net ultrafiltration.

A condition of edema is measured by the patient himself/herself checking an indentation. Specifically, this measurement is made by pressing a site where there is a bone such as a tibia under the skin with a thumb or the second to fourth fingers, and the condition of the skin after the finger is released is checked by visual inspection and palpation. The edema condition of "0" represents a normal condition without any indentation. The edema condition of "1+" represents a condition in which, although there is a slight indentation, there is no visible distortion, and the indentation disappears quickly. The edema condition of "2+" represents a condition in which there is a rather deep indentation but the indentation disappears within 10 to 15 seconds or so. The edema condition of "3+" represents a condition in which there is a deep indentation that may last 1 minute or longer. The edema condition of "4+" represents a condition in which there is a very deep indentation lasting 2 to 5 minutes or longer. In the patient information input screen in Fig. 5, the conditions of edema are input under three classifications of conditions of "0 to 1+", "2+", and "3+ to 4+". Alternatively, the conditions of edema may be input under five classifications of conditions "0" to "4+", respectively.

The urine output is measured by the patient himself/herself using a measurement cup or the like. In the patient information input screen in Fig. 5, a urine output (mL) per day is input. The net ultrafiltration is calculated as a drain volume subtracted from a fill volume. The fill volume herein represents the volume of dialysate infused into the body of the subject, and is measured by measuring the weight of the bag containing the dialysate before and after the infusion. The drain volume represents the volume of dialysate drained from the body of the subject, and is measured by measuring the weight of the bag storing the drainage. In the patient information input screen in Fig. 5, the net ultrafiltration (mL) per day is input.

Although the patient information input screen for receiving the information related to edema, a urine output, and a net ultrafiltration is illustrated in Fig. 5, the patient information input screen may also be configured to receive any one or two of edema, a urine output, and a net ultrafiltration.

Furthermore, information on mouth dryness or a body weight may be received via the patient information input screen. The mouth dryness is a subjective evaluation made by the patient, evaluated using a numerical rating scale (NRS) of 0 to 10. Alternatively, mouth dryness may be a binary evaluation: mouth dryness yes/mouth dryness no. The body weight is measured by the patient himself/herself using a scale.

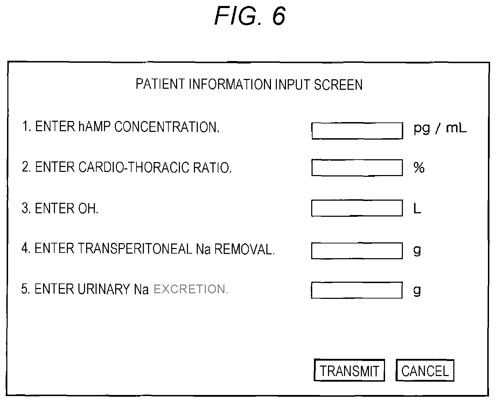

Furthermore, some of the patient information may be entered by a medical worker. Fig. 6 is a schematic illustrating an example of a patient information input screen on the doctor's terminal 30. The patient information input screen illustrated in Fig. 6 illustrates an example of a screen displayed on the display unit 35 when there is an access to the server device 10 from the doctor's terminal 30 and a predetermined operation is performed. The patient information input screen receives information related to hANP, a cardio-thoracic ratio, a bioelectrical impedance, a transperitoneal sodium removal, and a urinary sodium excretion.

The hANP stands for human atrial natriuretic peptide, and a concentration thereof is measured by a blood test. For the concentration measurement, an immunoradiometric assay (IRMA) is used. The reference value of hANP is, for example, 43.0 pg/mL. Because hAMP is secreted by being stimulated by atrial stretch, it shows an abnormally high value under the presence of diseases and pathological conditions causing an increase in the atrial pressure and an increase in the volume of a body fluid. In the screen example in Fig. 6, the hAMP concentration is entered as a numerical value (pg/mL).

The cardio-thoracic ratio is an index representing the ratio of the lateral cardiac diameter with respect to the lateral thoracic diameter, and is measured using a chest radiograph. 50% or less for men and 55% or less for women are considered appropriate. When the amount of extracellular fluid increases, the water content inside the blood vessel increases and the size of the heart also increases, so that the cardio-thoracic ratio becomes higher. In the screen example in Fig. 6, the cardio-thoracic ratio is entered as a numerical value (%).

The bioelectrical impedance is a resistance (impedance) of a living body, and is measured by a body composition analyzer. The body composition analyzer uses bioelectrical impedance analysis (BIA), which uses the property that adipose tissues pass almost no electrical current but lean tissues containing water and electrolyte easily pass electrical current. With the use of such BIA, the body composition analyzer can measure an index (OH) of excess/deficiency of a body fluid. The reference OH value is -1.1 L to 1.1 L. In the screen example in Fig. 6, the index of the body fluid excess/deficiency is entered as a numerical value (L).

The transperitoneal sodium removal is measured by collecting dialysate (waste liquid) discharged from the patient's body during peritoneal dialysis, and subjecting the waste liquid thus collected to a component analysis, for example. In the screen example in Fig. 6, a salt-conversion of the transperitoneal sodium removal is entered as a numerical value (g).

The urinary sodium excretion is measured by collecting urine of the patient, and subjecting the collected urine to a component analysis, for example. In the screen example in Fig. 6, a salt-conversion of the urinary sodium excretion is entered as a numerical value (g).

Although the patient information input screen for receiving the information related to the hANP, the cardio-thoracic ratio, the bioelectrical impedance, the transperitoneal sodium removal, and the urinary sodium excretion is illustrated in Fig. 6, the patient information input screen may be configured to receive any one or two or more of these pieces of information. Furthermore, it is not necessary to receive all of these pieces of information via one input screen, and individual pieces of information may be received individually.

Furthermore, information related to at least one of an antihypertensive agent, a diuretic, a blood pressure, urinary cortisol, urinary creatinine, a skin sodium content, and Na-MRI may be received via the patient information input screen.

The patient information input on the patient's terminal 20 and the doctor's terminal 30 is transmitted to the server device 10 via the communication network NW. The server device 10 generates an image representing the condition of a body fluid of the patient, on the basis of the patient information acquired from the patient's terminal 20 or the doctor's terminal 30. In this embodiment, information related to the condition of a body fluid is derived from the patient information using the learning model LM, and an image representing the condition of the patient's body fluid is generated on the basis of the derived information.

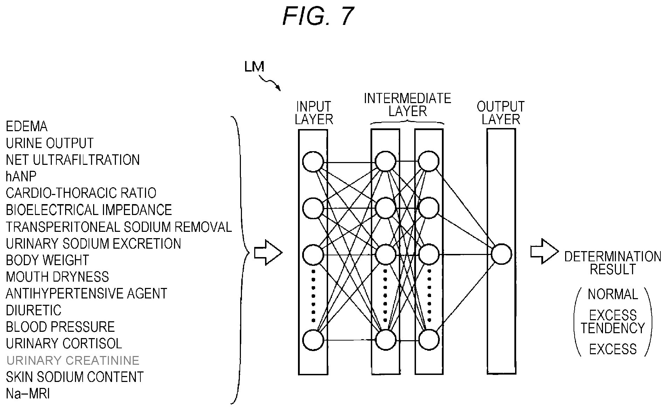

Fig. 7 is a schematic diagram illustrating a configuration example of the learning model LM. The learning model LM is trained to output information related to the condition of the body fluid of a patient, in response to an input of the patient information. As the learning model LM, a known learning model such as a convolutional neural network (CNN), a recurrent neural network (RNN), long short-term memory (LSTM), or an autoencoder is used.

The learning model LM includes an input layer, an intermediate layer, and an output layer. Each of these layers includes one or more nodes. The patient information is input to the nodes of the input layer. The information related to at least one of edema, a urine output, and a net ultrafiltration is input to the nodes of the input layer. The information input to the nodes of the input layer may further include at least one of pieces of information of hANP, a cardio-thoracic ratio, a bioelectrical impedance, a transperitoneal sodium removal, and a urinary sodium excretion. Furthermore, the information input to the nodes of the input layer may include information related to at least one of a patient's body weight, mouth dryness, an antihypertensive agent, a diuretic, and a blood pressure. Furthermore, the information input to the nodes of the input layer may include information related to at least one of urinary cortisol, urinary creatinine, a skin sodium content, and Na-MRI.

The intermediate layer of the learning model LM includes one or more layers. Each of the nodes in each of the layers included in the intermediate layer is coupled to the nodes belonging to the layers prior to or subsequent thereto. The activation level of each node and the strength of coupling (weighting coefficient) between the nodes are determined in the course of training. Each node in each of these layers outputs a value calculated on the basis of the weighting coefficient and the activation level, to a next node. The output layer calculates a probability using a softmax function on the basis of the values input from the intermediate layer, and outputs information based on the calculated probability. For example, the output layer calculates a probability of the body fluid volume being normal, a probability of the body fluid volume tending to be excessive, and a probability of the body fluid volume being excessive, and outputs information that is based on the probabilities. For example, the learning model LM may output information for determining whether the patient's body fluid volume is normal, tends to be excessive, or is excessive, in response to an input of the patient information. Such a learning model LM is generated by training an existing learning algorithm using a data set including patient information such as edema, a urine output, and a net ultrafiltration obtained for a patient, and an actual measurement (ground-truth value) of the body fluid volume measured using a measuring instrument such as a body composition analyzer as training data.

It is assumed that the trained learning model LM has been stored in the storage unit 12 of the server device 10. Upon acquiring patient information from the patient's terminal 20 or the doctor's terminal 30, the control unit 11 of the server device 10 inputs the acquired patient information to the learning model LM, causes the learning model LM to execute computation, and acquires the computation result from the learning model LM. In this embodiment, information for determining whether the volume of the body fluid of the patient is normal, tends to be excessive, or is excessive is acquired as the result of the computation of the learning model LM.

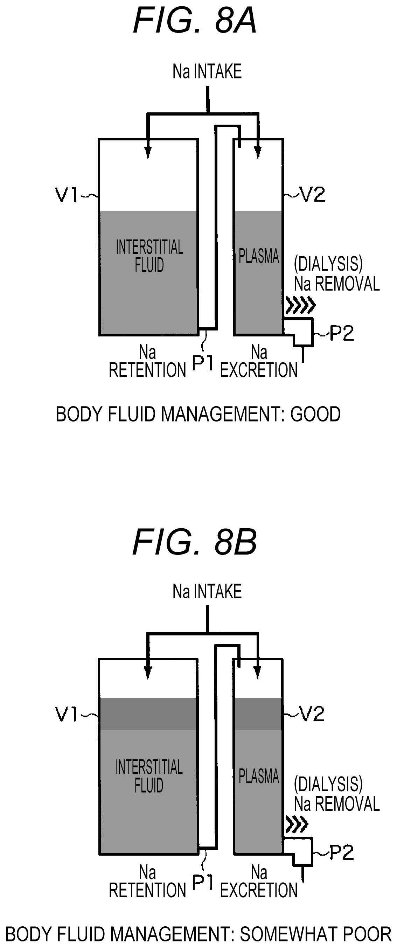

The control unit 11 of the server device 10 generates an image representing the condition of the body fluid of the patient on the basis of the result of the computation of the learning model LM. Figs. 8A to 8C are schematics illustrating examples of the generated image. Each of these images shows illustrations of a first vessel V1 storing therein interstitial fluid, a second vessel V2 storing plasma, a passage P1 connecting the first vessel V1 and the second vessel, and an excretion path P2 through which the liquid from the second vessel is drained. The interstitial fluid stored in the first vessel V1 and the plasma stored in the second vessel V2 can circulate from one vessel to the other vessel through the passage P1. The amount of sodium excreted as urine is illustrated as the amount of liquid through the excretion path P2. Also, the amount of sodium removed by dialysis is represented by the number of ">" symbols.

Fig. 8A shows that the sodium has been sufficiently removed by peritoneal dialysis and the body fluid is well-managed. Upon receiving a determination result that the volume of the body fluid of the patient is normal from the learning model LM, the control unit 11 generates and outputs an image, such as that illustrated in Fig. 8A. The receiver of the output may be either the patient's terminal 20 or the doctor's terminal 30.

Fig. 8B shows that sodium removed by the peritoneal dialysis is insufficient and that the body fluid management is somewhat poor. Upon receiving a determination result that the volume of the body fluid of the patient tends to be excessive from the learning model LM, the control unit 11 generates an image with slightly higher volumes of the interstitial fluid and the plasma, as illustrated in Fig. 8B, and outputs the image to the patient's terminal 20 or the doctor's terminal 30.

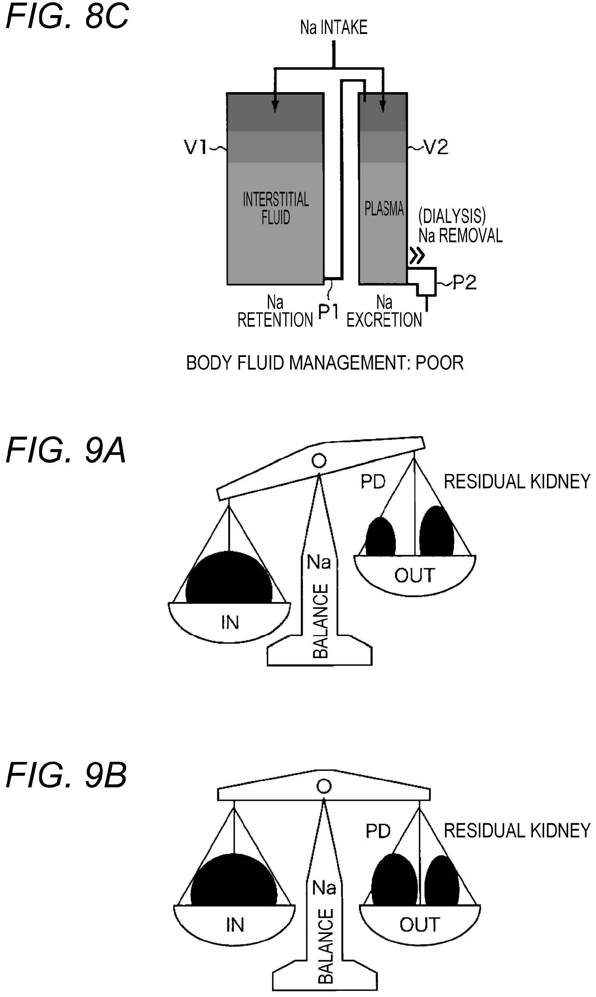

Fig. 8C shows that the sodium is not well removed by peritoneal dialysis, and the body fluid is poorly managed. Upon receiving a determination result that the volume of the body fluid of the patient is excessive from the learning model LM, the control unit 11 generates an image in which the first vessel V1 and the second vessel V2 are filled with the interstitial fluid and the plasma, respectively, as illustrated in Fig. 8C, and outputs the image to the patient's terminal 20 or the doctor's terminal 30.

The examples of Figs. 8A to 8C show the images in which the amount of sodium excreted as urine as well as the amount of sodium removed by dialysis are illustrated, in addition to the volume of the body fluid (volumes of the interstitial fluid and the plasma) of the patient, but it is also possible to generate an image in which only the volume of the body fluid is illustrated, by omitting the illustration of the amount of sodium.

Upon receiving an image such as those illustrated in Figs. 8A to 8C from the server device 10, the patient's terminal 20 displays the received image on the display unit 25. The patient can then understand his/her condition of the body fluid intuitively by checking the image displayed on the display unit 25.

Upon receiving an image such as those illustrated in Figs. 8A to 8C from the server device 10, the doctor's terminal 30 displays the received image on the display unit 35. The medical worker can get a grasp of the conditions of the body fluid of the patient by checking the image displayed on the display unit 35, and use it as a community tool for giving an instruction on the appropriate body fluid management.

Figs. 9A and 9B are schematics illustrating a modification of the image generated by the server device 10. Figs. 9A and 9B schematically illustrate the balance between the amount of sodium intake and the amount of sodium discharge. These schematics indicate that, even if the amount of sodium intake and the amount of sodium excreted as urine by the residual kidney function (residual kidney) are substantially constant, the Na balance is lost when there is a change in the amount of sodium removed by peritoneal dialysis (PD).

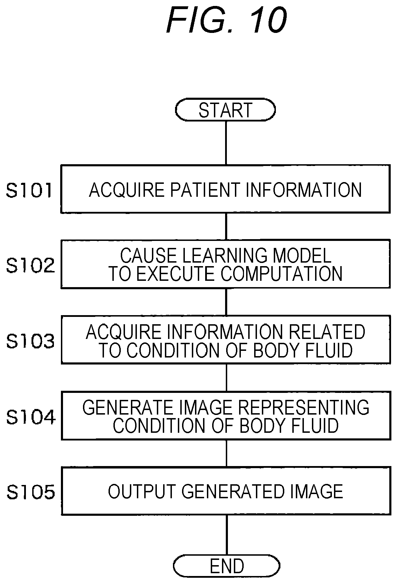

Fig. 10 is a flowchart for explaining the sequence of processing executed by the server device 10 according to this embodiment. The server device 10 acquires patient information from the patient's terminal 20 or the doctor's terminal 30 via the communication network NW (step S101). The patient information acquired by the server device 10 includes information related to at least one of edema, a urine output, and a net ultrafiltration. In addition to these pieces of information, information such as hANP, a cardio-thoracic ratio, a bioelectrical impedance, a transperitoneal sodium removal, and a urinary sodium excretion may be acquired.

The control unit 11 of the server device 10 inputs the acquired patient information to the learning model LM, and causes the learning model LM to perform the computation (step S102). The control unit 11 acquires information related to the condition of the body fluid of the patient, as the result of the computation of the learning model LM (step S103).

The control unit 11 generates an image representing the condition of the body fluid of the patient on the basis of the acquired information related to the condition of the body fluid of the patient (step S104). The control unit 11 may generate an image, as illustrated in Figs. 8A to 8C, by changing the amounts of the interstitial fluid in the first vessel V1 and the plasma in the second vessel V2 depending on whether the volume of the body fluid is normal, tends to be excessive, or is excessive. In addition, as illustrated in Figs. 8A to 8C, the control unit 11 may generate images in which sodium is removed by different amounts by dialysis, respectively, depending on the quantities of the interstitial fluid and the plasma. Alternatively, the control unit 11 may generate an image, such as that illustrated in Figs. 9A to 9B, schematically illustrating the balance between the amounts of sodium intake and sodium discharge.

The control unit 11 transmits the generated image to the patient's terminal 20 or the doctor's terminal 30 (step S105). The image transmitted from the server device 10 reaches the patient's terminal 20 or the doctor's terminal 30 through the communication network NW. Upon receiving the image transmitted by the server device 10, the patient's terminal 20 displays the received image on the display unit 25. Upon receiving the image transmitted by the server device 10, the doctor's terminal 30 displays the received image on the display unit 35.

As described above, in this embodiment, the condition of a body fluid of a patient can be presented to the patient himself/herself or to a medical worker. By checking the image displayed on the display unit 25 of the patient's terminal 20, the patient can understand the condition of his/her body fluid, by feel. By checking the image displayed on the display unit 35 of the doctor's terminal 30, the medical worker can get a grasp of the condition of the body fluid of the patient and use it as a community tool for giving an instruction on the appropriate body fluid management.

It should be construed that the embodiment disclosed herein is illustrative in all respects rather than restrictive. The scope of the present invention is defined by the claims, and not by the meaning above, and is intended to include all changes that fall within the meaning and scope of the claims and equivalents thereof.

For example, in this embodiment, the server device 10 is configured to cause the learning model LM to execute the computation, and to generate the image using the result of the computation of the learning model LM, but each of the patient's terminal 20 and the doctor's terminal 30 may be configured to cause the learning model LM to execute the computation, and to generate the image using the result of the computation of the learning model LM. In such a case, the image generation program PG and the learning model LM are installed on each of the patient's terminal 20 and the doctor's terminal 30.

Furthermore, in this embodiment, the patient information is input to a learning model LM to acquire the information related to the condition of a body fluid, and an image representing the condition of the body fluid of the patient is generated on the basis of the acquired information. However, the image representing the condition of a body fluid of a patient may be generated directly from the patient information using a generative learning model for images, such as Generative Adversarial Network (GAN) or Progressive Growing of GANs (PGGAN).

### Reference Signs List

- 10: server device
- 11: control unit
- 12: storage unit
- 13: communication unit
- 14: operation unit
- 15: display unit
- 20: patient's terminal
- 21: control unit
- 22: storage unit
- 23: communication unit
- 24: operation unit
- 25: display unit
- 30: doctor's terminal
- 31: control unit
- 32: storage unit
- 33: communication unit
- 34: operation unit
- 35: display unit
- PG: image generation program
- LM: learning model

## Claims

1. A computer program for causing a computer to execute processing comprising:
acquiring information on a peritoneal dialysis patient, the information including information on at least one of edema, a urine output, and a net ultrafiltration;
generating an image representing a condition of a body fluid of the peritoneal dialysis patient on a basis of the information thus acquired; and
outputting the image thus generated.

2. The computer program according to claim 1, wherein the information further includes information related to at least one of a human atrial natriuretic peptide (hANP), a cardio-thoracic ratio, a bioelectrical impedance, a transperitoneal sodium removal, and a urinary sodium excretion.

3. The computer program according to claim 2, wherein the information further includes information related to at least one of a body weight, mouth dryness, an antihypertensive agent, a diuretic, and a blood pressure of the peritoneal dialysis patient.

4. The computer program according to claim 3, wherein the information further includes information related to at least one of urinary cortisol, urinary creatinine, a skin sodium content, and nuclear magnetic resonance (Na-MRI).

5. The computer program according to claim 1, further causing the computer to execute processing of generating an image schematically representing a sodium retention and a sodium excretion of the peritoneal dialysis patient, as the image.

6. The computer program according to claim 5, further causing the computer to execute processing of generating an image including a first vessel storing interstitial fluid and a second vessel storing plasma, as the image, wherein
the image represents the sodium retention as an amount of interstitial fluid in the first vessel.

7. The computer program according to claim 6, further causing the computer to execute processing of
generating an image including a passage through which the interstitial fluid and the plasma flow between the first vessel and the second vessel, and an excretion path through which sodium is excreted as urine from the second vessel,
the image being an image schematically representing the sodium excretion on a basis of an amount of sodium removed from the plasma by peritoneal dialysis and the amount of sodium excreted through the excretion path, as the image.

8. The computer program according to claim 1, further causing the computer to execute processing of generating an image schematically representing a balance between a sodium intake and a sodium excretion of the peritoneal dialysis patient, as the image.

9. The computer program according to any one of claims 1 to 8, further causing the computer to execute processing of:
inputting acquired information on the peritoneal dialysis patient to a learning model having been trained to output information on a condition of the body fluid in response to an input of the information on at least one of edema, a urine output, and a net ultrafiltration, and causing the learning model to execute computation; and
generating an image representing the condition of the body fluid of the peritoneal dialysis patient on a basis of a result of the computation of the learning model.

10. An image output method that uses a computer to execute processing comprising:
acquiring information on a peritoneal dialysis patient, the information including information on at least one of edema, a urine output, and a net ultrafiltration;
generating an image representing a condition of a body fluid of the peritoneal dialysis patient on a basis of the information thus acquired; and
outputting the image thus generated.

11. An image output device comprising at least one processor, wherein the processor
acquires information on a peritoneal dialysis patient, the information including information on at least one of edema, a urine output, and a net ultrafiltration;
generates an image representing a condition of a body fluid of the peritoneal dialysis patient on a basis of the information thus acquired; and
outputs the image thus generated.
